Europäisches Patentamt

European Patent Office

Office européen des brevets

(11) Publication number: **0 220 650**

**A2**

## (12) EUROPEAN PATENT APPLICATION

(21) Application number: 86114523.3

(22) Date of filing: 20.10.86

(51) Int. Cl.⁴: **C12M 3/00**

(30) Priority: **21.10.85 US 789649**

(43) Date of publication of application:
**06.05.87 Bulletin 87/19**

(84) Designated Contracting States:
**AT BE CH DE ES FR GB GR IT LI LU NL SE**

(71) Applicant: **ENDOTRONICS INC.**
**8500 Evergreen Boulevard**
**Coon Rapids Minnesota 55433(US)**

(72) Inventor: **Martinez, Jesus F.**
**2625 Jewel Lane**
**Plymouth Minnesota 55447(US)**
Inventor: **Martin, William R.**
**3511 Humboldt Avenue North**
**Minneapolis Minnesota 55412(US)**
Inventor: **Gruenberg, Micheal L.**
**5900 Boulder bridge Lane**
**Shorewood Minnesota 55331(US)**

(74) Representative: **Vossius & Partner**
**Siebertstrasse 4 P.O. Box 86 07 67**
**D-8000 München 86(DE)**

(54) Method and device for culturing cells.

㊄ A method for culturing cells includes introducing cells within an extracapillary space defined by a shell and a plurality of hollow semi-permeable fibers extending therethrough. The semi-permeable fibers are spaced from each other a distance substantially similar to a distance that tissue capillaries are spaced from each other in in vivo living tissue. Media is passed through the hollow fibers and cell growth having a density substantially equal to that of in vivo living tissue is observed.

EP 0 220 650 A2

# METHOD AND DEVICE FOR CULTURING CELLS

The present invention relates to methods for cell culturing, and in particular, it relates to culturing cells using a plurality of substantially equally spaced hollow fibers disposed within a shell for the growth and maintenance of cells in vitro.

Culturing cells in vitro in devices having a plurality of randomly arranged hollow fibers disposed within a shell in a bundle has been known for quite some time. Media containing oxygen, nutrients and other chemical stimuli is transported through lumens of the hollow fibers. Cells are grown in a cell culturing space that is defined between the exterior of the hollow fibers and the interior wall of the shell.

Culturing devices having hollow fibers have proven to be suitable for the maintenance of many types of cells in vitro. The mass transfer characteristics of hollow fibers provide an efficient means of delivering nutrients to and removing waste products from a cell culture. Hollow fibers with an appropriate pore size are selected to maintain product produced by the cells within the cell culturing space while waste products pass through the fibers' walls and into the lumen of the fibers.

As the cells grow, multiply and secret product and with the product being maintained in the cell culturing space, the secreted product becomes concentrated. The production of a concentrated product gives hollow fiber culture device great potential for economic production of cell-derived products on a commercial scale.

However, the prior art hollow fiber culture devices are self-limiting with respect to cell density due to very poor distribution of the hollow fiber within the cell culturing space. Ordinarily, the prior art contains many examples of hollow fiber devices where the cell culturing space is filled by a plurality of fibers arranged in a loosely and axially oriented bundle where individual fibers are next to one another, blocking a large percentage of the cell culturing space. In the presence of this random fiber arrangement, cells will tend to grow on fibers which are located on the outside of the bundle since the inside of the bundle does not allow enough space for cell growth. As this situation develops in a cell culture, a significant portion of the nutrients are distributed to areas of the culturing space with very low or nonexistent cell growth causing poor nutrient supply and waste removal from areas with high density growth.

Some examples of prior art hollow fiber culturing devices are described in the following US-patents:

Patent No.

3,734,851

3,821,087

3,883,393

3,911,140

3,997,396

4,087,327

4,184,922

4,200,689

4,201,845

--continued--

Patent No.

4,206,015

4,220,725

4,242,460

4,391,912

4,396,510

4,440,853

4,442,206

The following US-patents illustrate the structure of hollow fiber cartridges used in dialysis. Although the dialysis cartridges described in the following patents are somewhat suitable for use as cell culturing devices, they are not designed to meet the needs of cells growing in a high density environment.

Patent No.

4,141,835

4,227,295

4,276,687

4,283,284

4,226,378

3,492,698

3,442,002

4,176,069

Commercially available dialysis cartridges are also described in brochures distributed by Erika Inc., a division of National Medical Care of Rockleigh, New Jersey, and CD Medical, Inc. of Miami Lakes, Florida.

The present invention includes a method for culturing cells in a device having hollow fibers spaced apart generally the same distance as tissue capillaries are spaced apart in _in vivo_ living tissue. The cell culturing device includes a shell and a plurality of hollow fibers extending through the shell for passage of a media. The hollow fibers have selectively semi-permeable walls for permitting passage of nutrients and chemical stimuli to the cells and permitting passage of waste into the hollow fibers. A cell culturing space for the growing and maintenance of living cells is defined between an inner wall of the shell and the exterior of the hollow fibers.

The hollow fibers are arranged in a manner providing for efficient transfer of nutrients and chemical stimuli and providing for efficient manner of removal of wastes. Preferably, the hollow fibers are connected to each other by sewn thread such that the distance from one hollow fiber to the next hollow fiber is generally the same as the distance from one blood capillary to the next blood capillary in _in vivo_ living tissue. Preferably, this distance is in the approximate range of 25 to 200 microns.

Figure 1 is an elevational view of the cell culturing device of the present invention.

Figure 2 is an end view of the device in Figure 1.

Figure 3 is a cross sectional view of the device taken along the line 3--3 in Figure 2.

Figure 4 is a plan view of a section of hollow fibers illustrating the spacing between the hollow fibers from each other caused by sewn thread.

Figure 5 is an enlarged cross sectional view of the device taken along the line 4--4 in Figure 3.

Figure 6 is an enlarged cross sectional view of several layers of hollow fibers illustrating the spacing between individual hollow fibers caused by the sewn thread.

Figure 7 is a graph comparing daily glucose consumption by an IgG producing hybridoma cell line between the bioreactor of the present invention and two commercially available bioreactors.

Figure 8 is a graph comparing the daily lactic acid production of the IgG producing hybridoma cell line between the bioreactor of the present invention and two commercially available bioreactors.

Figure 9 is a graph illustrating the concentration of IgG in daily samples between the bioreactor of the present invention and two commercially available bioreactors.

Figure 10 is a graph illustrating the total production of IgG per day between the bioreactor of the present invention and two commercially available bioreactors.

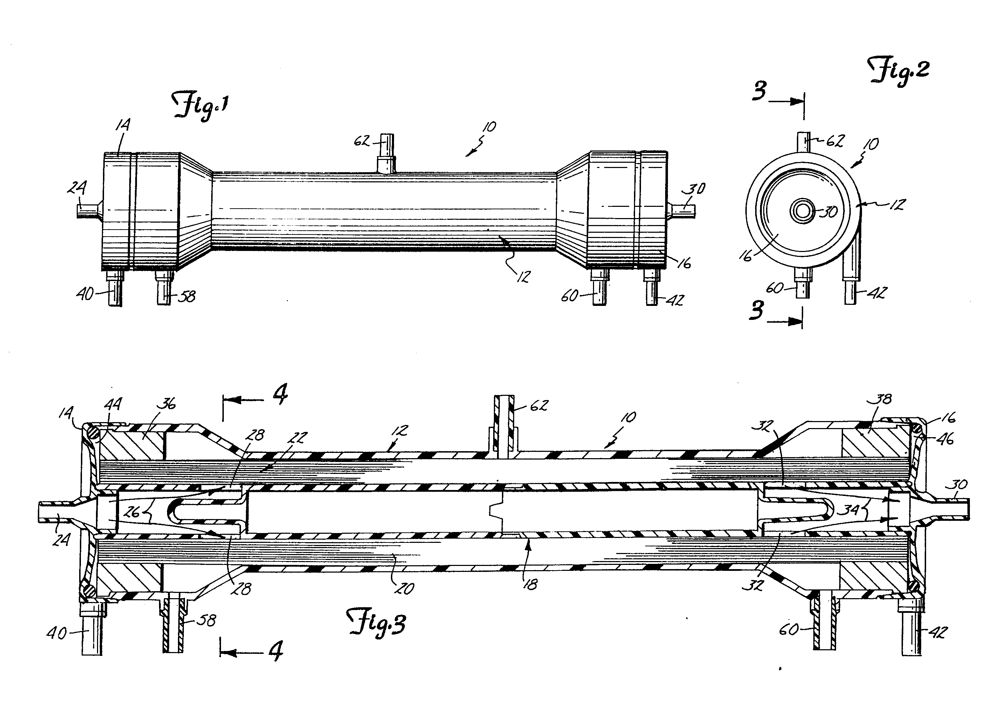

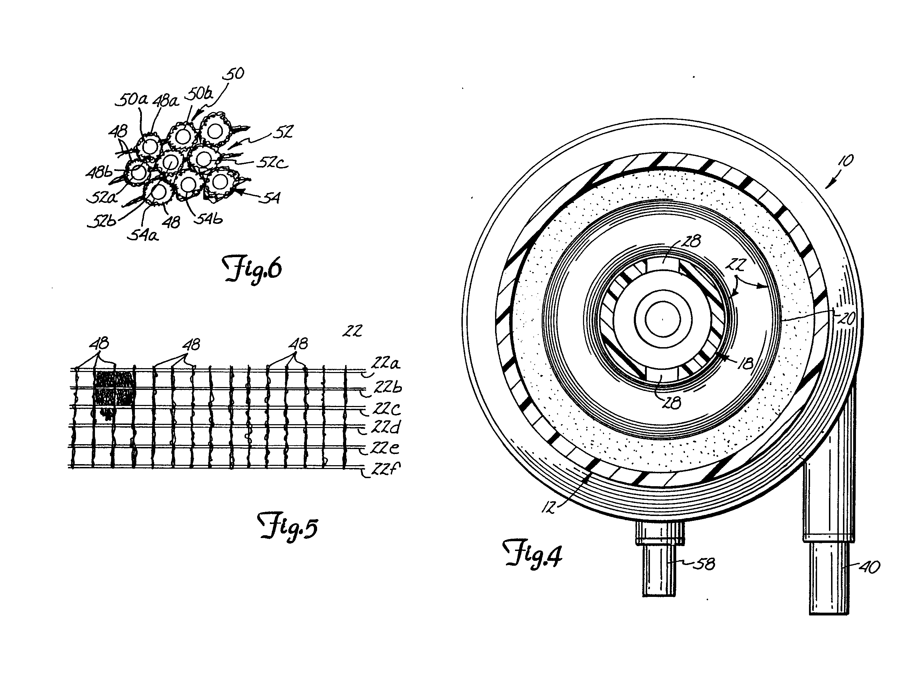

A bioreactor of the present invention is generally indicated at 10 in Figures 1 and 2. The bioreactor 10 includes an outer shell 12 having end cap portions 14 and 16. An inner core 18 is disposed generally coaxially within the shell 12, as illustrated in Figure 3. A plurality of hollow fibers, generally indicated at 22, are disposed within the shell and extend from one end of the shell to another. A cell culturing space 20 is defined between the core 18 and the shell 12 exteriorly of the fibers.

The end cap 14 includes an inlet port 24 that permits the flow of media in a general direction of arrows 26 into the cell culturing space 20 through openings 28 in the core 18. The port 24 is used primarily to innoculate the culturing space 20 with a cell line. Flow through port 24 is generally stopped once the cell line has been established in the space 20.

The end cap 16 includes an outlet port 30. The port 30 permits media to flow out of the space 20 by flowing through openings 32 in the core 18 as generally indicated by arrows 34. The flow of nutrient solution into port 24, entering the space 20, and exiting the space 20 through port 30 may be stopped after innoculation, or may be continued to supply nutrients or chemical stimuli to the cells in the cell culturing space 20.

The fibers 22 are potted in a potting compound as indicated by reference characters 36 and 38 at opposite ends of the shell 12 as is well known in the art. The cap 14 includes an inlet port 40 and the cap 16 includes an outlet port 42. The inlet and outlet ports 40 and 42 are preferably disposed tangentially, as described in the Schäel et al U.S. Patent 4,141,835, which is herein incorporated by reference. As also described in the Schäel et al Patent, a ring-shaped manifold chamber 44 is defined between the end cap 14 and the potting compound 36 and is fluidly connected to the port 40. The chamber 44 is used to supply the media in a uniform manner to the lumens of the hollow fibers. Likewise, a ring-shaped chamber 46 is defined between the end cap 16 and the potting compound 38 and is fluidly connected to the outlet port 42. The port 42 provides an outlet for media leaving the hollow fibers 22. It will be understood by those skilled in the art that the outlet port 42 can be used as an inlet and the inlet port 40 can be used as an outlet by simply reversing the flow through the fibers.

The hollow fibers are spaced from each other substantially the same distance as tissue capillaries, such as blood capillaries, are spaced from each other in living tissue. It is believed that cells which constitute in vivo living tissue are no more than approximately 100 microns from a· tissue capillary or other similar vessel. It has been found that by spacing the individual hollow fibers a maximum of approximately 200 microns from each other, cell densities are achieved that equal the cell densities of in vivo living tissue. Further, it has been found that a range of spacing between fibers is satisfactory, the range being from 25 to 200 microns with 100 microns being especially preferred.

Preferably, the hollow fibers 22 are spaced from each other by a plurality of spaced-apart sewn threads 48, as illustrated in Figure 4. Connecting hollow fibers by sewn threads to form a type of mat and then winding the mat of hollow fibers around a core is well known in the construction of dialysis cartridges. In addition, dialysis cartridges having fibers spaced in the preferred range of 25-200 microns are commercially available. One such cartridge is available from Erika Inc., a division of National Medical Care of Rockleigh, New Jersey. However, the present invention contemplates using such spacing for the culturing of cells which has not been known. It has been found by the applicants that the spacing of the hollow fibers from each other is critical to the efficient transfer of nutrients and the efficient removal of waste from cells located at a position furthest from the hollow fibers. To this end, the individual fibers, as illustrated in Figure 4, and identified by reference characters 22a through 22f, are spaced apart and held in a spaced-apart manner by the sewn threads 48 a distance of approximately 100 microns.

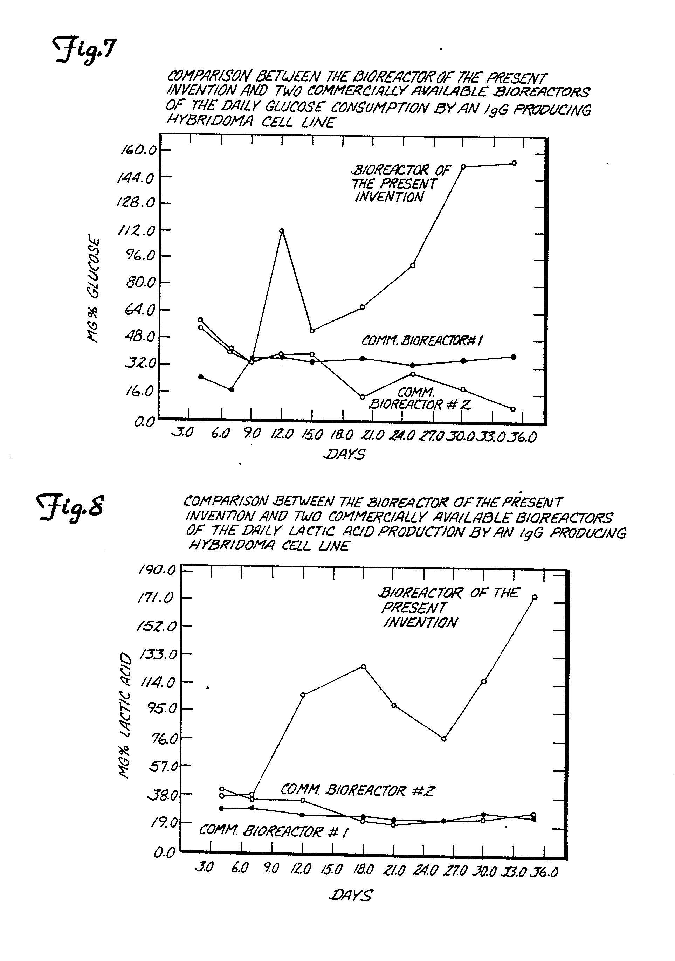

As illustrated in Figure 5, wherein a cross sectional view of the cell culturing device is illustrated, the sewn mat of fibers is wound around the central core 18 until the hollow fibers fill substantially the entire cell culturing space 20 in a spiral-type fashion. In Figure 6, a three-layer portion of the spirally wound mat of hollow fibers is shown in an enlarged cross section. The layers are designated by the reference characters 50, 52 and 54. It will be understood that all three layers are preferably part of the same hollow fiber mat. The layers 50, 52, and 54 are spaced and separated from each other in a generally radial direction approximately 100 microns from each other by the sewn thread 48. The sewn thread 48 is formed by two individual threads 48a and 48b sewn together in a lock stitch. Each individual hollow fiber, such as hollow fiber 50a, is held within the sewn thread by one thread 48a surrounding one half the circumference of the hollow fiber 50a and the other thread 48b surrounding the other half of the hollow fiber 50a. The threads 48a and 48b are then joined together for an approximate distance of 100 microns before being separated again to retain the next hollow fiber 50b.

The threads 48a and 48b also separate the hollow fibers layers 50, 52 and 54 from each other. The threads 48a and 48b are of a thickness such that each hollow fiber, for example, hollow fiber 52b is positioned and retained approximately 100 microns from hollow fibers 50a, 50b, 54a and 54b. The axial connection of the fibers by the sewn thread 48 and the separation of the layers of fibers from each other creates a three-dimensional network of hollow fibers such that no cell is more than 100 microns from a hollow fiber surface. The result is that even the furthest cell will receive a sufficient amount of nutrients in a high cell density environment. In addition, waste is efficiently removed, minimizing deleterious effects on cell growth or production of product from the cells. A high cell density similar to cell density found in in vivo living tissue has been observed.

To further encourage better nutrient transfer and waste removal within the cell culturing space 20, the bioreactor of the present invention includes circulation ports 58, 60 and 62, as illustrated in Figures 1 and 3. The ports 58, 60 and 62 are fluidly connected to the cell culturing space 20. Preferably, the port 58 is located at one end of the shell proximate the potting compound 36 and the port 60 is located at an opposite end of the shell proximate the potting compound 38, while the port 62 is located about mid-way on the shell, 180° from the ports 58 and 60. The port 62 may be used as an inlet and the ports 58 and 60 may be used as outlets, or the flow may be reversed. Circulating a media containing a nutrient within the cell culturing space is discussed in patent applications by Cracauer et al, Serial No. 658,549 filed on October 9, 1984 entitled "Improved Hollow Fiber Culture Device and Method of Operation"; and Walker et al, Serial No. 658,550 filed on October 9, 1984 entitled "Hollow Fiber Culture Device for Improved Nutrient Perfusion and Product Concentration and Method of Operation", both applications assigned to the assignee of the present application and are hereby incorporated by reference.

EXAMPLE

Methods and Materials

A woven fiber bioreactor assembly of the present invention with cuprophan hollow fibers was compared with two commercial bioreactors. One of the commercial bioreactors contains saponified cellulose acetate fibers, while the second commercial bioreactor contains cuprophan hollow fibers similar to the bioreactor of the present invention. Both commercial bioreactors had follow fibers arranged randomly in a bundle. The bioreactor of the present invention did not include the circulation port arrangement, that is three ports; as described previously. The port arrangement 58, 60 and 62 were not included so that a valid comparison of fiber spacing between the bioreactor of the present invention and the two commercial bioreactors would be valid. The two commercial bioreactors will be referred to as commercial bioreactor #1 and commercial bioreactor #2. Each bioreactor was flushed with one liter of sterile distilled water and 500 mls of cell culture medium before use, to remove wetting agents and other water soluble compounds. A medium reservoir was placed in line using silicone tubing between each bioreactor and a peristaltic pump. A commercially available medium was pumped through the fibers of the bioreactors at a flow rate of 200 mls/minute using the peristaltic pump. Fresh medium was pumped into the reservoir at a rate sufficient to maintain a pH in the system of 7.0 and used medium removed at about the same rate. The system was maintained at a temperature of 37°C. A gas exchange cartridge was placed in line between the bioreactors and the medium reservoir and connected to a filtered source of 10% carbon dioxide and 90% air. A syringe was used to introduce four hundred million cells of an IgG producing hybridoma cell line into each bioreactor through the sample ports. Once a week, 10 mls of fetal bovine serum were injected into the extracapillary spaces containing the cells. Three times a week, samples were taken from the extracapillary spaces and the medium reservoir in order to monitor the pH and the pressures of oxygen and carbon dioxide, using a Blood Gas Analyzer. The glucose, lactic acid, and antibody concentrations were also monitored using standard assay procedures. After about two weeks, a continuous harvest was initiated involving the removal of one ml/hour of media from the extracapillary spaces and assayed for antibody concentrations.

Results

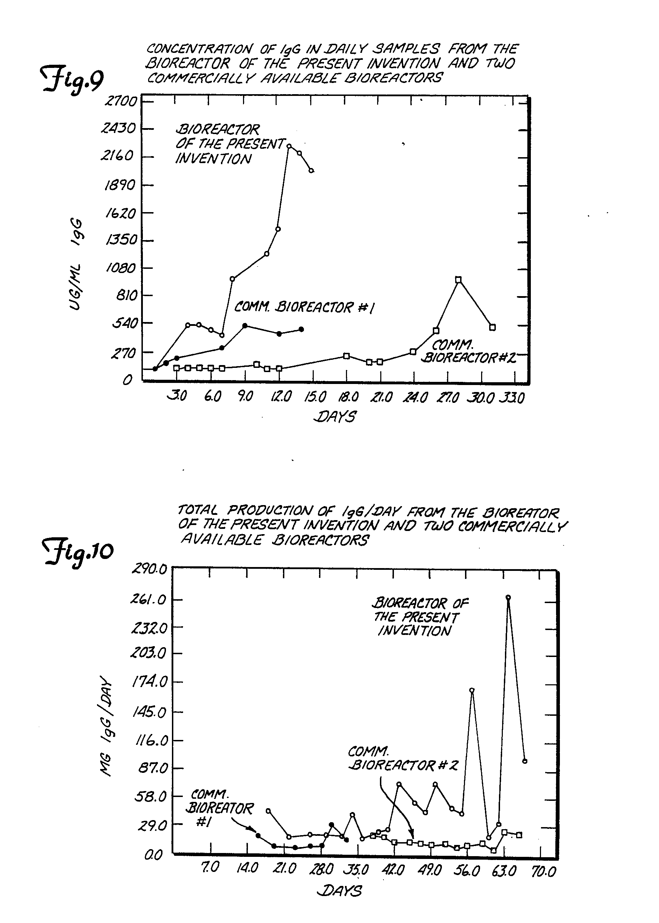

The growth and metabolism of the cells in the bioreactors was monitored through the measurement of three parameters: glucose consumption, lactic acid production, and antibody production. Glucose consumption was very similar in all three bioreactors for the first nine days. After nine days, the cells in the woven fiber assembly of the present invention began consuming glucose at a far faster rate, and at the end of 35 days, glucose consumption in the woven fiber assembly of the present invention was approximately five times greater than the glucose consumption in either of the commercial bioreactors, as illustrated in Figure 7. The production of lactic acid followed a very similar pattern, production was approximately the same in all three bioreactors for the first nine days and then began to increase rapidly in the woven fiber assembly, as illustrated in Figure 8. At the end of 35 days, the lactic acid production in the woven fiber assembly was more than five times greater than that in the commercial bioreactors. The production of antibody in the woven fiber assembly was also consistently higher, running anywhere from two to eight times as great as that being produced in the commercial bioreactors, as illustrated in Figures 9 & 10.

Conclusion

All three parameters, glucose consumption (as illustrated in Figure 7), lactic acid production (as illustrated in Figure 8), and antibody production (as illustrated in Figures 9 & 10), indicated very strongly that the bioreactor of the present invention was superior to either of the commercial bioreactors for the support of cellular growth, metabolism, and the production of antibody. In addition, the daily visual examination of the bioreactors found cell density in the woven fiber assembly to be strikingly greater than that of the commercial bioreactors.

Although the present invention has been described with reference to preferred embodiments, workers skilled in the art will recognize that changes may be made in form and detail without departing from the spirit and scope of the invention.

**Claims**

1. A method for the maintenance of living cells in vitro comprising:
introducing living cells into a cell culturing space of a bioreactor having a plurality of hollow fibers extending within the cell culturing space, the hollow fibers spaced from each other a distance substantially similar to the spacing of tissue capillaries found in in vivo living tissue; and
passing media through the hollow fibers.

2. The method of claim 1 wherein the hollow fibers are spaced from each other by connecting the hollow fibers to each other by a plurality of sewn threads.

3. The method of claim 2 including arranging the fibers within the cell culturing space on a core such that the fibers are substantially parallel with each other.

4. The method of claim 3 including wrapping the hollow fibers around the core in a spiral fashion resulting in spiral layers of the hollow fibers.

5. The method of claim 4 and including spacing the nearest hollow fiber in each spiral layer from the nearest hollow fiber in an adjacent spiral layer approximatly 25 to 200 microns.

6. The method of claim 4 and including spacing the hollow fibers within each spiral layer from an adjacent hollow fiber in the same spiral layer approximately 25 to 200 microns.

7. The method of claim 1 and further including introducing media through the cell culturing space from a source exterior of the bioreactor such that circulation of the media is effected within the cell culturing space.

8. The method of claim 1 and further including spacing the hollow fibers from each other approximately 25 to 200 microns.

9. The method of claims 5, 6 and 8 and including spacing the hollow fibers from adjacent hollow fibers approximately 100 microns.

10. A bioreactor device having a shell and a plurality of semi-permeable hollow fibers extending therethrough for passage of media and a cell culturing space defined within the shell for the maintenance of living cells or microorganisms, the device comprising:

the hollow fibers being arranged and retained with respect to each other a distance substantially similar to a distance that tissue capillaries found in in vivo living tissue are spaced from each other; and

means for introducing media into the cell culturing space through the shell such that circulation is effected in the cell culturing space.

11. The device of claim 10 wherein the means for introducing media into the cell culturing space includes first and second fluid ports arranged on the shell such that circulation is effected substantially within the entire cell culturing space.

12. The device of claim 10 wherein the means for introducing media into the cell culturing space includes first, second and third fluid ports with the first and second fluid ports being disposed proximate opposite ends of the shell and fluidly communicating with the cell culturing space and the third port being positioned approximately mid-way between the first and second ports and 180° angularly from the first and second ports.

13. The device of claim 10 wherein the hollow fibers are connected to each other by a plurality of sewn threads forming a mat and the mat being wound in a spiral within the cell culturing space.

14. The device of claim 13 wherein each spiral layer is separated from the adjacent spiral layer by the sewn thread such that each hollow fiber is separated from an adjacent hollow fiber, whether an adjacent hollow fiber within the same layer or an adjacent hollow fiber in an adjacent layer, approximately 25 to 200 microns.

15. The device of claim 14 wherein each hollow fiber is separated from an adjacent hollow fiber approximately 100 microns.

*Fig.1*

*Fig.2*

*Fig.3*

Fig.4

Fig.6

Fig.5

## Fig.7

COMPARISON BETWEEN THE BIOREACTOR OF THE PRESENT
INVENTION AND TWO COMMERCIALLY AVAILABLE BIOREACTORS
OF THE DAILY GLUCOSE CONSUMPTION BY AN IgG PRODUCING
HYBRIDOMA CELL LINE

## Fig.8

COMPARISON BETWEEN THE BIOREACTOR OF THE PRESENT
INVENTION AND TWO COMMERCIALLY AVAILABLE BIOREACTORS
OF THE DAILY LACTIC ACID PRODUCTION BY AN IgG PRODUCING
HYBRIDOMA CELL LINE

**Fig.9**

CONCENTRATION OF IgG IN DAILY SAMPLES FROM THE BIOREACTOR OF THE PRESENT INVENTION AND TWO COMMERCIALLY AVAILABLE BIOREACTORS

**Fig.10**

TOTAL PRODUCTION OF IgG/DAY FROM THE BIOREATOR OF THE PRESENT INVENTION AND TWO COMMERCIALLY AVAILABLE BIOREACTORS